(19) Europäisches Patentamt
European Patent Office
Office européen des brevets

(11) **EP 1 114 017 B1**

(12) # EUROPÄISCHE PATENTSCHRIFT

(45) Veröffentlichungstag und Bekanntmachung des Hinweises auf die Patenterteilung:
**19.03.2003 Patentblatt 2003/12**

(21) Anmeldenummer: **99942870.9**

(22) Anmeldetag: **13.08.1999**

(51) Int Cl.[7]: **C07C 45/50**, B01J 10/00, B01J 19/24, B01J 19/26

(86) Internationale Anmeldenummer:
**PCT/EP99/05967**

(87) Internationale Veröffentlichungsnummer:
**WO 00/009467 (24.02.2000 Gazette 2000/08)**

(54) **VERFAHREN ZUR HERSTELLUNG VON ALDEHYDEN UND/ODER ALKOHOLEN ODER AMINEN**
METHOD FOR PRODUCING ALDEHYDES AND/OR ALCOHOLS OR AMINES
PROCEDE DE PREPARATION D'ALDEHYDES ET/OU D'ALCANOLS OU D'AMINES

(84) Benannte Vertragsstaaten:
**AT BE CH CY DE DK ES FI FR GB GR IE IT LI LU MC NL PT SE**

(30) Priorität: **14.08.1998 DE 19836807**

(43) Veröffentlichungstag der Anmeldung:
**11.07.2001 Patentblatt 2001/28**

(73) Patentinhaber: **BASF AKTIENGESELLSCHAFT
67056 Ludwigshafen (DE)**

(72) Erfinder:
- **ZEHNER, Peter
D-67071 Ludwigshafen (DE)**
- **ULONSKA, Armin
D-67112 Mutterstadt (DE)**
- **PACIELLO, Rocco
D-67098 Bad Dürkheim (DE)**

(56) Entgegenhaltungen:
**EP-A- 0 087 670**
**DE-A- 3 220 858**
**GB-A- 2 222 098**
**DE-A- 2 645 780**
**DE-A- 4 427 428**
**US-A- 4 216 339**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von Aldehyden und/oder Alkoholen oder gegebenenfalls Aminen durch die Umsetzung von Olefinen mit Kohlenmonoxid und Wasserstoff in An- oder Abwesenheit von Ammoniak oder einem primären oder sekundären Amin in Gegenwart eines homogen im Reaktionsmedium löslichen Katalysators, enthaltend mindestens ein Element ausgewählt aus Kobalt, Rhodium oder Ruthenium in An- oder Abwesenheit eines Phosphor-, Arsen-, Antimon- oder Stickstoff- enthaltenden Liganden bei erhöhter Temperatur und bei erhöhtem Druck unter Verwendung eines Düsen-Umlauf-Reaktors.

Mit Hilfe der Hydroformylierung von Olefinen werden weltweit jährlich etwa 7 Millionen Tonnen verschiedener Produkte hergestellt. Dabei handelt es sich um Aldehyde, Alkohole oder Amine. Aldehyde werden im wesentlichen durch die Hydroformylierung von Olefinen mit homogen im Reaktionsmedium löslichen Kobaltcarbenylverbindungen oder Rhodium- oder Rutheniumcarbonylkomplexen, in der Regel Rhodiumcarbonylkomplexen, die mit einem phosphor-, arsen, antimon- oder stickstoff enthaltenden Liganden in ihrer Reaktivität und Selektivität modifiziert sind, erzeugt.

Unter Hydroformylierung versteht man die Umsetzung von Olefinen mit $H_2$/CO-Gemischen, gemeinhin als Synthesegas bezeichnet, zu Aldehyden gemäß Gleichung (1)

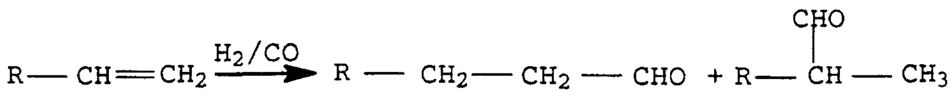


(1)

in Gegenwart eines Katalysators aus der VIII Nebengruppe des Periodensystems der Elemente. Dem Bereich der Hydroformylierungsreaktionen zuzuordnen sind weiterhin die sogenannte hydrierende Hydroformylierung, bei der der im Hydroformylierungsschritt gebildete Aldehyd in situ im Hydroformylierungsreaktor vom Hydroformylierungskatalysator zum entsprechenden Alkohol hydriert wird, sowie die sogenannte aminierende Hydroformylierung. Obgleich auch heterogenisierte Hydroformylierungskatalysatoren eingesetzt werden können, hat sich in der industriellen Anwendung der Hydroformylierungsreaktion die Verwendung von homogen im Hydroformylierungsmedium löslichen Komplexen dieser Elemente durchgesetzt. Üblicherweise werden Kobalt-, Rhodium-, Palladiumoder Rutheniumcarbonylverbindungen verwendet, wobei diese Verbindungen in ihrer Reaktivität und Chemoselektivität durch Komplexierung mit phosphor-, arsen-, antimon- oder stickstoff enthaltenden Liganden bevorzugt werden.

Die Hydroformylierung wird üblicherweise bei erhöhter Temperatur durchgeführt, wobei die bevorzugten Temperaturbereiche je nach Art des eingesetzten Hydroformylierungskatalysators variieren können. Je nach eingesetztem Hydroformylierungskatalysator und dem Druckbereich, in dem dieser bevorzugt in der Technik eingesetzt wird, wird generisch zwischen 3 Typen der Hydroformylierung unterschieden, nämlich der Hochdruckhydroformylierung, die bei Temperaturen von im allgemeinen 140 bis 200°C und bei einem Druck von 100 bis 600 bar durchgeführt wird, der Mitteldruckhydroformylierung, die im allgemeinen bei Temperaturen von 120 bis 180°C und im Druckbereich von 30 bis 100 bar ausgeübt wird und die Niederdruckhydrofomylierung, bei der im allgemeinen Temperaturen von 60 bis 130°C und ein Druck von 1 bis 30 bar angewendet wird.

Im allgemeinen werden für diese verschiedenen Hydroformylierungsverfahren vorzugsweise unterschiedliche Katalysatoren eingesetzt, und zwar im Hochdruckverfahren unter den angewandten Reaktionsbedingungen nicht mit zusätzlichen organischen Liganden modifizierte Carbonylverbindungen oder Hydrocarbonylverbindungen, vorzugsweise des Kobalts oder Rhodiums, die sich unter den Hydroformylierungsbedingungen aus leicht zugänglichen Vorläuferverbindungen bilden, bei der Mitteldruckhydroformylierung mit phosphorhaltigen Liganden, insbesondere Phosphinliganden modifizierte Kobaltcarbonylkomplexe und bei der Niederdruckformylierung vorzugsweise Rhodiumcarbonylkomplexe mit vorzugsweise phosphorhaitigen Liganden, insbesondere mit Phosphin- oder Phosphitliganden.

Die einzelnen in den verschiedenen Hydroformylierungsverfahren eingesetzten Katalysatoren unterscheiden sich nicht nur in ihrer Hydroformylierungsaktivität sondern auch in ihrer Chemoselektivität, d.h. in ihrer Eigenschaft ein bestimmtes der in Gleichung (1) dargestelltes isomeren Hydroformylierungsprodukte bevorzugt zu bilden und in ihrer Eigenschaft außer der Hydroformylierungsaktivität noch weitere katalytische Aktivitäten besitzen, die je nach dem zu hydroformylierenden Olefin und dem gewünschten Hydroformylierungsprodukt mehr oder weniger erwünscht sein können.

So besitzen viele der bekannten Hydroformylierungskatalysatoren zusätzlich eine je nach den angewandten Reaktionsbedingungen nicht unbeträchtliche Hydrieraktivität und zwar sowohl für C-C-Doppelbindungen als auch für die C-O-Doppelbindungen der Carbonylgruppe. Während die Nebenreaktion der C-O-Doppelbindung in der Regel unerwünscht ist, da sie zur Bildung geringwertiger Paraffine führt, kann die Hydrierung der Carbonylgruppen der im Zuge der Hydroformylierung gebildeten Aldehyde zu den betreffenden Alkoholen durchaus erwünscht sein, da sich hierdurch eine gegebenenfalls erforderliche zusätzliche Hydrierstufe erübrigt. Eine solche Hydrieraktivität der Hydroformylierungskatalysatoren ist beispielsweise auch bei der aminierenden Hydroformylierung von Olefinen erwünscht, wobei das aus dem entstehenden Aldehyd und einem im Reaktionsmedium vorliegenden primären bzw. sekundären Amin gebildete Imin bzw. Enamin in situ zum gewünschten Amin hydriert wird.

Eine andere katalytische Nebenaktivität mancher Hydroformylierungskatalysatoren ist die Isomerisierung von Doppelbindungen, beispielsweise von Olefinen mit internen Doppelbindungen zu α-Olefinen und umgekehrt.

Mit phosphorhaltigen Liganden modifizierte Kobaltcarbonylkomplexe besitzen z.B. nicht nur Hydroformylierungsaktivität sondern sind zusätzlich als Hydrierkatalysatoren sehr wirksam, weshalb je nach dem angewandten CO/$H_2$-Verhältnis im zur Hydroformylierung eingesetzten Synthesegas, die bei der Hydroformylierung der Olefine mit solchen Kobaltkatalysatoren gebildeten Aldehyde ganz oder teilweise zu den entsprechenden Alkoholen hydriert werden, so daß je nach den angewandten Reaktionsbedingungen Alkohole oder Aldehyd-/Alkohol-Gemische entstehen (vgl. B. Cornils in J. Falbe, New Syntheses with Carbon Monoxide, Springer Verlag, Berlin [1980] S. 1-181)

Diese Nebenaktivitäten der Hydroformylierungskatalysatoren können in ihrem Ausmaß zum Teil durch die Einstellung bestimmter Hydroformylierungsbedingen im Reaktionsmedium in der gewünschten Weise beeinflußt werden. Allerdings können oftmals schon geringe Abweichungen von den für das jeweilige Ausgangsolefin und das gewünschte Hydroformylierungsprodukt optimierte Verfahrensparameter zur Bildung erheblicher Mengen an unerwünschten Nebenprodukten führen, weshalb die Einstellung praktisch identischer Verfahrensparameter über das Volumen der gesamten Reaktionsflüssigkeit im Hydroformylierungsreaktor von erheblicher Bedeutung für die Wirtschaftlichkeit des Verfahrens sein kann. Dies gilt aufgrund ihrer verfahrenstechnischen Besonderheiten insbesondere für Mitteldruck- und Niederdruckhydroformylierungsverfahren, deren Verbesserung der Gegenstand der vorliegenden Erfindung ist.

Bei der Hydroformylierung beispielsweise eines α-Olefins können, je nach dem an welchem Kohlenstoffatom der olefinischen Doppelbindung sich das Kohlenmonoxid anlagert, geradkettige, sogenannte n-Aldehyde, oder verzweigte, sogenannte iso-Aldehyde, gebildet werden (siehe Gleichung 1). Beispielsweise bilden sich bei der Hydroformylierung von Propen n-Butylaldehyd und Isobutylaldehyd. Für die jeweiligen n- und iso-Produkte, die bei der Hydroformylierung bestimmter Olefine erhalten werden, besteht seitens des Marktes eine unterschiedliche Nachfrage. Man ist deshalb bestrebt, diese isomeren Aldehyde in einem bestimmten n/iso-Verhältnis zu erzeugen, das dem Bedarf für die einzelnen Isomere entspricht. Das n/iso-Verhältnis kann in gewissem Umfang durch die Einstellung bestimmter Reaktionsparameter im Hydroformylierungsreaktor beeinflußt werden.

Im allgemeinen ist es bei der Hydroformylierung von Olefinen mit Hilfe ligandmodifizierter Homogenkatalysatoren vorteilhaft, eine optimale Konzentration an im flüssigen Reaktionsmedium gelöstem Wasserstoff und Kohlenmonoxid einzustellen, wobei insbesondere der Konzentration an gelöstem Kohlenmonoxid, nachstehend auch mit der Abkürzung [CO] bezeichnet, eine besondere Bedeutung zukommt. Insbesondere bei der Niederdruckhydroformylierung von Olefinen mit Phosphinligand-modifizierten Rhodiumcarbonylkomplexen verursachen bereits geringfügige Abweichungen der Konzentration an gelöstem Kohlenmonoxid der optimalen [CO] eine Verschlechterung des Ergebnisses der Hydroformylierung. Die Beeinflussung des Ergebnisses der Niederdruckhydroformylierung von Olefinen durch unterschiedliche Reaktionsparameter wurde wissenschaftlich am eingehensten für die Hydroformylierung mit Rhodiumcarbonyl-Triphenylphosphin (TPP)-Komplexen untersucht, weshalb dies nachstehend am Beispiel dieser Hydroformylierungskatalysatoren, stellvertretend für andere Ligand-modifizierte, in der Niederdruckhydroformylierung angewandte oder anwendbare Katalysatoren, erläutert wird.

Das n/iso-Verhältnis des bei der Hydroformylierung mit Rh/TPP-Katalysatoren erzeugten Aldehydprodukts wird maßgeblich von dem Verhältnis der Kohlenmonoxid- und Triphenylphospin-Konzentration [CO]/[TPP] in der Reaktionsflüssigkeit beeinflußt. So besteht nach ausführlichen Untersuchungen von Cavalieri d'Oro et al (La Chimica e l'Industria <u>62</u>, 572 (1980) ein hyperbolischer Zusammenhang zwischen dem [CO]/[TPP]-Verhältnis und dem n/iso-Verhältnis, wobei zur Erzielung eines hohen n/iso-Verhältnis im Bereich des aufsteigenden Astes der Hyperbel gearbeitet werden muß. Wenn das [CO]/[TPP]-Verhältnis erniedrigt wird, steigt die Selektivität für die Bildung des in vielen Fällen besonders erwünschten n-Aldehyds. Eine Erniedrigung dieses [CO]/[TPP]-Verhältnisses kann bewirkt werden, indem man den Partialdruck $P_{CO}$ des Kohlenmonoxids in der Gasphase des Hydroformylierungsreaktors absenkt und/oder die TPP-Konzentration erhöht. Dabei ist allerdings zu berücksichtigen, daß die durch den hydrieraktiven Rh-TPP-Katalysator als Nebenreaktion katalysierte Paraffinbildung zunimmt, wenn der CO-Partialdruck und damit auch die Konzentration an gelöstem CO in der Reaktionsflüssigkeit abnimmt und die Reaktionsgeschwindigkeit verlangsamt wird, wenn die TPP-Konzentration zu hoch wird.

Die Paraffinbildung, die Bildung hochsiedender Kondensationsprodukte der gebildeten Aldehyde, sogenannte Hochsieder, als auch die Standzeit des Rh-TPP-Katalysators werden zudem durch die Reaktionstemperatur beeinflußt. Für die optimale Durchführung von Hydroformylierungen im technischen Maßstab, wo großvolumige Reaktoren mit einer Kapazität von über 100 000 t/Jahr nicht ungewöhnlich sind, kommt es deshalb im entscheidenden Maße darauf an, daß sich über das Volumen der im Reaktor befindlichen Reaktionsflüssigkeit keine Gradienten bezüglich der Reaktionstemperatur und der Konzentration an gelöstem CO ausbilden, d.h. daß über das gesamte Flüssigkeitsvolumen identische, für die Erzeugung eines gewünschten n/iso-Verhältnis optimale Betriebsbedingungen einstellen lassen.

Auf diese Sachverhalte wird explizit auch in DE-A 2810644, EP-A 254 180, US-A 4 277 627, EP-A 188 246, EP-A 423 769 und WO 95/08525 hingewiesen.

Während bei der Niederdruckhydrofomylierung mit Phosphinligand-modifizierten Rhodiumcarbonylkomplexen das [CO]/[TPP]-Verhältnis von besonderer Bedeutung für das Hydroformylierungsergebnis ist, gilt dies bei der Mitteldruckhydroformylierung mit Phosphinligand-modifizierten Kobaltcarbonylkomplexen auch für das Verhältnis von gelöstem Kohlenmonoxid und Wasserstoff, wohingegen bei Phospitligand-modifizierte Rhodiumcarbonylkomplexe, wie sie ebenfalls bei der Niederdruckhydroformylierung angewendet werden, empfindlich auf die Überschreitung des optimalen Temperaturbereichs bei der Hydroformylierung reagieren können.

Um die Investitions- und Betriebskosten für eine Hydroformylierungsanlage möglichst niedrig zu halten, aber auch aus Sicherheitsgründen, ist man bestrebt, den volumetrischen Gasanteil eG bei einer bestimmten Temperatur und einem bestimmten Druck, der durch Gleichung (1) definiert ist

$$e_G \frac{\text{Gasvolumen in der Flüssigkeit}}{\text{Gesamtvolumen von Gas und Flüssigkeit im 2-Phasensystem}}$$

zu minimieren und so die Raum-Zeit-Ausbeute (RZA) zu maximieren. Unter RZA wird dabei die pro Zeit- und Volumeneinheit umgesetzte Menge an Olefinen, bezogen auf das Gesamtvolumen des Reaktors, verstanden. Es ist anhand des vorstehenden leicht verständlich, daß bei einem hohen volumetrischen Gesamtteil $e_G$ in der Reaktionsflüssigkeit, die RZA abnimmt, da die Hydroformylierungsreaktion in der flüssigen Phase stattfindet und das überschüssige Gasvolumen in der Reaktionsflüssigkeit wertvollen Reaktionsraum nutzlos beansprucht. Da sich im technischen Betrieb des Hydroformylierungsverfahrens die chemische Zusammensetzung der Reaktionsflüssigkeit aufgrund der Bildung der Hydroformylierungsprodukte und Nebenprodukte, wie Hochsiedern verschiedener chemischer Zusammensetzung ändert und das Aufnahmevermögen solcher Reaktionsmischungen für die dispergierten Blasen des Reaktionsgases nicht an jedem Betriebspunkt bekannt ist, kann es in Extremfällen zu einem Überlaufen des Hydroformylierungsreaktors aufgrund eines zu hohen volumetrischen Gesamtteils in der Reaktionsflüssigkeit kommen, zu dessen Vermeidung der Hydroformylierungsreaktor ingenieurmäßig mit einem größeren Volumen ausgelegt wird, als dies aus Kapazitätsgründen erforderlich wäre. Umgekehrt führt eine Verarmung der Reaktionsflüssigkeit an gelöstem Kohlenmonoxid als Folge der Ausbildung von [CO]-Gradienten im Volumen der Reaktionsflüssigkeit aufgrund einer ungleichmäßigen Gasdurchmischung zu lokalen Umsatzeinbußen im Reaktor und somit ebenfalls zu einer Verringerung der RZA sowie zu einer Erhöhung der Paraffinbildung.

Zur Verhinderung der Ausbildung von Konzentrationsgradienten und Temperaturinhomogenitäten in der Reaktionsflüssigkeit ist eine intensive Durchmischung der Reaktionsflüssigkeit erforderlich, weshalb deren ideale Durchmischung angestrebt wird. In EP-A 188 246, EP-A 423 769 und WO 95/08525 wird zu diesem Zweck die Verwendung von Rührern oder Begasungsrührern oder die Nutzung der in die Reaktionsflüssigkeit eingeleiteten Reaktionsgasströme zur Durchmischung der Reaktionsflüssigkeit vorgeschlagen. Zur Verteilung des Reaktionsgases werden Gasverteiler eingesetzt, deren Größe, Anzahl und Lage im Reaktor von der Größe des Reaktors abhängt. Die Abführung der Reaktionswärme erfolgt mit Hilfe interner, im Reaktor befindlicher oder externer Wärmeaustauscher.

Die in EP-A 188 246, EP-A 423 769 und WO 95/08525 vorgeschlagenen Reaktoren haben die gemeinsamen Nachteile, daß sich der volumetrische Gasanteil in der Reaktionsflüssigkeit praktisch nur über eine Erhöhung oder Erniedrigung der durch die Reaktionsflüssigkeit geleiteten Gasströme verändern läßt. Eine gezielte Regelung von $e_G$ durch die Veränderung der Rührerdrehzahl ist zwar prinzipiell möglich, aber wenig effektiv.

Angesichts der vorstehenden Angaben versteht es sich von selbst, daß zur Erzielung einer optimalen Durchmischung der Reaktionsflüssigkeit in einem großvolumigen Hydroformylierungsreaktor mit einer Produktionskapazität von z.B. 100 000 t/Jahr mechanisch sehr aufwendig gebaute Rührwerkskonstruktionen eingesetzt werden müssen, die entsprechend teuer in der Anschaffung sind. Aus diesem Grund wird in der Technik oftmals die Alternative, mehrere kleinere Rührkessel anstelle eines einzigen großen Reaktors einzusetzen, ergriffen. Diese Alternative hat ebenfalls einen erhöhten Investitionsaufwand zur Folge. Ein weiterer Nachteil der Verwendung von Rührern zur Durchmischung der Hydroformylierungsflüssigkeit besteht darin, daß die Rührerwelle durch die Wandung des Druckreaktors geführt werden und diese Durchführung mit Lagern und Dichtungen versehen werden muß, die einer erheblichen Belastung ausgesetzt sind und in relativ kurzer Zeit verschleißen. Dergleichen sind die Rührerblätter oder Rotoren einer hohen mechanischen Beanspruchung ausgesetzt. Zur Auswechslung dieser Verschleißteile muß der Reaktor abgeschaltet werden.

Da die Hydroformylierungsreators stark exotherm ist und deren Selektivität wie vorstehend dargelegt gegenüber Konzentrationsgradienten und Temperaturinhomogenitäten im flüssigen Reaktionsmedium sensitiv ist, muß für eine gute Durchmischung der Reaktionsflüssigkeit gesorgt werden, andernfalls die Gefahr besteht, daß der stabile Betriebsbereich des Reaktors, beispielsweise durch lokale Überhitzungen, verlassen wird, mit der Folge von Ausbeute- und Selektivitätsverlusten.

Während eine Erhöhung oder Erniedrigung der Rührerdrehzahl eine Veränderung der Durchmischung der

Reaktionsflüssigkeit bewirkt, hat eine Erhöhung oder Erniedrigung der Rührerdrehzahl nur eine vergleichsweise geringe Auswirkung auf den volumetrischen Gasanteil $e_G$ der Reaktionsflüssigkeit. Somit ist die Veränderung der Rührerdrehzahl keine geeignete Maßnahme zur Regelung des volumetrischen Gasanteils $e_G$ in der Reaktionsflüssigkeit ist.

Als Alternative zu Rührreaktoren werden zur Durchführung der Hydroformylierungsreaktion in der Industrie Blasensäulen eingesetzt. Hierbei werden die Reaktionsgase am unteren Ende der Blasensäule über einen Gasverteiler, der für die Dispergierung der Reaktionsgase in der Reaktionsflüssigkeit zwecks Erhöhung der Stoffaustauschfläche sorgt, eingeführt. Die feinen Gasbläschen steigen entsprechend ihrer geringeren Dichte in der Reaktionsflüssigkeit nach oben, wodurch die Reaktionsflüssigkeit durchgemischt wird. Während des Aufsteigens diffundiert ein Teil der Gase aus den Gasbläschen durch die Grenzfläche Gas/Flüssigkeit in die Reaktionsflüssigkeit, worin sie in gelöster Form in der Hydroformylierungsreaktion abreagieren. Wird die Blasensäule durch eine entsprechend eingestellte Zufuhr der Reaktionsgase bei einem relativ niedrigen volumetrischen Gasanteil $e_G$ betrieben, kommt es über die Länge der Flüssigkeitssäule in der Blasensäule zur Ausbildung von [CO]-Konzentrationsgradienten und Temperaturinhomogenitäten mit den beschriebenen nachteiligen Folgen auf Ausbeute und Selektivität. Bei einem zur idealen Durchmischung der Reaktionsflüssigkeit erforderlichen hohen volumetrischen Gasanteil, wird ein großer Teil des zur Verfügung stehenden Reaktionsraumes nutzlos von den Gasbläschen belegt, wodurch die RZA sinkt.

Zur Lösung des vorstehenden Problems wird in EP-A 254 180 vorgeschlagen, ein Teil der Reaktionsgase über verschiedene Zuführungen in unterschiedlicher Höhe des als Blasensäule ausgestalteten Reaktors in die Reaktionsflüssigkeit einzuleiten. Durch diese Maßnahme wird eine Verringerung des [CO]-Gradienten von üblicherweise 10 % auf 2 % abgesenkt.

DE-A 2810644 betrifft die Anwendung eines gefluteten Reaktors, d.h. eines Reaktors, dessen gesamtes Volumen von der Reaktionsflüssigkeit ausgefüllt wird, für Hydroformylierungsreaktionen, bei denen im unteren Teil des Reaktors die flüssigen und gasförmigen Reaktanten zugeführt und im Inneren eines rohrförmigen im Reaktor befindlichen Leitorgan, dessen unteres und oberes Ende jeweils in einem Abstand zum Reaktorboden bzw. zur Reaktorkappe gelegen sind, in den oberen Teil des Reaktors geleitet. Dort wird der aufwärts gerichtete Strom der Reaktionsflüssigkeit umgekehrt, so daß er im Zwischenraum zwischen der Wandung des Leitorgans und der Reaktorwandung abwärts strömt, wo er beim Auftreffen auf den Reaktorboden oder geeignete Umlenkvorrichtungen wieder in eine im Inneren des Leitorgans aufwärts fließende Strömung umgelenkt wird. Ein Teil des Reaktionsgemisches wird kontinuierlich zur Produktisolierung an einer Entnahmestelle im unteren Teil des Reaktors abgeführt, die so angebracht ist, daß praktisch nur Reaktionsflüssigkeit aus der abwärts fließenden Strömung entnommen wird. Die Volumina der Reaktorräume mit nach oben bzw. nach unten gerichteten Strömung sind etwa gleich groß. Dieses Verfahren läßt sich vorteilhaft bei der Hochdruckhydrofomylierung von Olefinen mit nicht - ligandmodifizierten Kobaltcarbonylen als Katalysatoren durchführen, da die n/iso-Selektivität dieser Kobaltcarbonyle praktisch nicht von der Konzentration des in der Reaktionsflüssigkeit gelösten CO beeinflußt wird. Der bei der Hochdruckhydroformylierung mit Kobaltcarbonylen angewandte Druck - weit über 100 bar - ist so groß, daß praktisch das gesamte, dem Reaktor zugeführte Kohlenmonoxid in der Reaktionsflüssigkeit gelöst vorliegt, wodurch es zu keiner CO-Verarmung der Reaktionsflüssigkeit kommt. Nur um eine grobe Vorstellung zu geben, sei hier erwähnt, daß die [CO] bei 20 bar und 100°C, also bei Bedingungen, wie sie bei der Rhodium-Niederdruckhydroformylierung mit Phosphinliganden typisch sind, in der Größenordnung von ungefähr 200 g $CO/m^3$ Reaktionsflüssigkeit liegt, wohingegen die [CO] bei 280 bar und 100°C im Bereich von Kilogramm $CO/m^3$ Reaktionsflüssigkeit liegt. Weiterhin ist die Reaktionsgeschwindigkeit bei der mit Kobaltcarbonylen katalysierten Hochdruckhydroformylierung um ein Vielfaches geringer als bei der mit Rhodiumcarbonyl-Ligand-Komplexen katalysierten Niederdruckhydroformylierung. Dementsprechend ist es auch das Ziel der Anwendung der in DE-A 2810644 beschriebenen Reaktorkonstruktion, die Verweilzeit der Reaktionsflüssigkeit durch die Verlängerung der von der Reaktionsflüssigkeit im Reaktor zurückzulegenden Wegstrecke zu erhöhen, um einen höheren Olefinumsatz zu erzielen. Die Vermeidung der Gradientenbildung wird in DE-A 2810644 nicht angesprochen. Da es sich bei der Vorrichtung von DE-A 2810644, bildlich ausgedrückt, praktisch um einen in der Mitte umgestülpten Rohrreaktor handelt, der beim Betrieb bei einem Reaktionsdruck, wie er in der Niederdruck- oder Mitteldruckhydroformylierung angewandt wird, einer in der Mitte umgestülpten Blasensäule entspricht, ist die Anwendung dieser Reaktorkonstruktion im Verfahren zur Niederdruck- oder Mitteldruckhydroformylierung mit den gleichen Problemen konfrontiert, wie sie bei der Anwendung von Blasensäulen auftreten und wie sie zuvor geschildert wurden.

In Vorarbeiten der Erfinder wurde nun gefunden, daß die Geschwindigkeit der Hydroformylierungsreaktion in einem weiten Parameterbereich kinetisch kontrolliert ist, d.h. die Geschwindigkeit des Stoffübergangs aus der Gasphase in die flüssige Phase wirkt sich unter den üblicherweise angewandten Hydroformylierungsbedingungen nicht limitierend auf die Reaktionsgeschwindigkeit der Hydroformylierung aus. Daraus folgt, daß mit abnehmendem volumetrischen Gasanteil $e_G$ die RZA steigt. Der volumetrische Gasanteil eG kann nun aber nicht beliebig abgesenkt werden, da irgendwann die Grenze überschritten wird, wo die Geschwindigkeit des Stoffübergangs aus der Gas- in die Flüssigphase limitierend für die Reaktionsgeschwindigkeit der Hydroformylierungsreaktion wird. Die Grenze zwischen kinetischer und Stoffübergangs-beeinflußter Kontrolle der Reaktionsgeschwindigkeit der Hydroformylierungsreakton verläuft fließend. Sie ist in komplexer Weise von der Art des mechanischen Leistungseintrags in die Reaktionsflüssigkeit, der spezifischen Phasengrenzfläche zwischen Gas und Reaktionsflüssigkeit, ausgedrückt in $m^2/m^3$, sowie den physikalischen Eigenschaften des Reaktionssystems abhängig. Eine exakte Vorausberechnung ist bisher noch nicht gelungen.

Beim Betrieb eines industriellen Reaktors steht der Betreiber nun vor dem Dilemma, daß einerseits die Einstellung eines hohen volumetrischen Gasanteil $e_G$ in der Reaktionsflüssigkeit zum Zwecke der Verhinderung der Gradientenbildung eine nicht optimale Ausnutzung des Reaktorvolumens und damit eine nicht optimale RZA zur Folge hat, andererseits, bei einem zu niedrigen volumetrischen Gasanteil $e_G$, die Gefahr der Gradientenbildung mit nachteiligen Auswirkungen auf die Ausbeute, die Selektivität und das n/iso-Verhältnis besteht, der im Falle der Verwendung eines Rührreaktors nur mit wirtschaftlich unbefriedigenden Gegenmaßnahmen begegnet werden kann. Analoges gilt für die Ausbildung von Temperaturgradienten über das Volumen der Reaktionsflüssigkeit.

Aufgabe der vorliegenden Erfindung war es nun, ein Verfahren zur Niederdruck- oder Mitteldruckhydroformylierung von Olefinen zur Verfügung zu stellen, das es ermöglicht die Hydroformylierung bei einem zur Erzielung einer optimalen RZA erforderlichen volumetrischen Gasanteil $e_G$ der Reaktionsflüssigkeit und der unter Zugrundelegung eines gewünschten n/iso-Verhältnisses, zur Erzielung einer optimalen Ausbeute und Selektivität erforderlichen Gasverteilung und Durchmischung der Reaktionsflüssigkeit kontrolliert durchzuführen, ohne daß dies mit den vorstehend geschilderten wirtschaftlichen Nachteilen der Hydroformylierungsverfahren der Standes des Technik verbunden wäre.

Dementsprechend wurde ein Verfahren zur Herstellung von Aldehyden und/oder Alkoholen oder weiger bevorzugt, gegebenenfalls zu Aminen durch die Umsetzung von Olefinen in flüssiger Phase mit Kohlenmonoxid und Wasserstoff, wobei ein Teil dieser Gase in Form von Gasblasen in der Reaktionsflüssigkeit dispergiert und ein anderer Teil in der Reaktionsflüssigkeit gelöst ist, in An- oder Abwesenheit eines primären oder sekundären Amins und in Gegenwart eines homogen in der Reaktionsflüssigkeit gelösten Kobalt-, Rhodium-, Palladium- oder Ruthenium- Carbonylkomplexes mit einem Phosphor- Arsen-, Antimon- oder Stickstoff enthaltenden Liganden bei erhöhter Temperatur und bei einem Druck von 1 bis 10 bar gefunden, das dadurch gekennzeichnet ist, daß man die Umsetzung in einem senkrecht angeordneten, röhrenförmigen Reaktor, umfassend einen Reaktorkörper und mindestens eine Umlaufleitung, vornimmt, einen Teil der Reaktionsflüssigkeit kontinuierlich über die Umlaufleitung mindestens einer im oberen Teil des Reaktorkörpers angebrachten Düse zuführt, der ein oben und unten offenens, durch parallele Wände abgegrenztes Leitorgan im Innern des Reaktors, sowie eine unterhalb der untern Öffnung des Leitorgans befindlichen Prallplatte zugeordnet ist, und mit dieser Düse in diesem Leitorgan einen abwärts gerichteten dispergierte Gasblasen enthaltenden Flüssigkeitsstrom erzeugt, der nach Verlassen des Leitorgans in eine im Zwischenraum zwischen der Wandung des Leitorgans und der Wandung des Reaktorkörpers aufwärts fließenden Strom umgelenkt wird, und am oberen Ende des Leitorgans vom Strahl der dem Leitorgan zugeordneten Düse in das Leitorgan gesaugt wird.

Entsprechend einer bevorzugten Ausführungsform wird die Umsetzung gemäß Fig. 1 in einem Reaktor 1 durchgeführt, umfassend den Reaktorkörper 2 und mindestens eine Umlaufleitung 3, sowie Zuleitungs- und Ableitungsvorrichtungen für die Reaktanten und den Reaktionsaustrag, wobei man aus dem Reaktorkörper 2, vorzugsweise aus einem Beruhigungsraum unterhalb der Prallplatte 7 über mindestens eine Umlaufleitung 3 kontinuierlich einen Teil der Reaktionsflüssigkeit entnimmt und wobei man mit mindestens einer im oberen Teil des Reaktorkörpers 2 befindlichen Düse 5 einen Strahl erzeugt der in mindestens einem im Reaktor angebrachten Leitorgan 6 eine abwärts gerichtete Strömung erzeugt, die nach auftreffen auf mindestens eine dem betreffenden Leitorgan oder den Leitorganen 6 zugeordnete Umlenkvorrichtung in Form einer Prallplatte 7 in eine im Zwischenraum 8 zwischen Reaktorköper 2 und dem betreffenden Leitorgan 6 aufwärts fließende Strömung umgelenkt wird, wobei in dem Reaktorkörper 2 mindestens ein Leitorgan 6 so angebracht ist, daß

a) dessen oberes Ende bei Betrieb des Reaktors 1 unterhalb des Flüssigkeitspegels 9 der im Reaktorkörper 2 befindlichen Reaktionsflüssigkeit liegt und mindestens eine diesem Leitorgan 6 zugeordnete Düse 5 sich in etwa konzentrischer Position bezüglich der Querschnittsfläche dieses Leitorgans 6 befindet,

b) dessen unteres Ende im Abstand über dem Reaktorboden 13 oder über mindestens einer dem Leitorgan zugeordneten Umlenkvorrichtung in Form einer Prallplatte 7 gelegen ist,

und das oder die Leitorgane 6 so dimensioniert sind, daß das Verhältnis aus der Innenquerschnittsfläche des oder der Summe der Innenquerschnittsflächen der Leitorgane 6 und der gesamten Innenquerschnittsfläche des Reaktorkörpers 2 0,03 bis 0,6 und das Verhältnis aus der Länge L mindestens eines einer Düse 5 zugeordneten Leitorgans 6 und dessen Innendurchmesser d 3 bis 40 beträgt und die mindestens eine Düse 5 im Kopfraum 10 des Reaktorkörpers 2 so angeordnet ist, daß deren Spitze 11 sich im Betriebszustand des Reaktors im Bereich oder unterhalb des Flüssigkeitspegels 8, jedoch im Abstand a vom oberen Ende des dieser Düse 5 zugeordneten Leitorgans 6 befindet.

Die Durchführung des erfindungsgemäßen Verfahrens und des dafür verwendeten Reaktors wird im folgenden beispielhaft anhand der Fig. 1 noch ausführlicher erläutert.

Der im erfindungsgemäßen Verfahren anwendbare Reaktor 1 umfaßt im allgemeinen einen druckstabilen Reaktorkörper 2 und mindestens eine Umlaufleitung 3. Die Querschnittsform des Reaktorkörpers 2 ist im Prinzip für die Durchführbarkeit des erfindungsgemäßen Verfahrens nicht kritisch und kann dreieckig, viereckig, quadratisch, rechteckig, rautenförmig, trapezförmig, fünfeckig, sechseckig, polygonal, elliptisch oder kreisförmig sein, vorzugsweise ist sie kreisförmig. Dem Reaktorkörper 2, der vor der Inbetriebnahme vorzugsweise bis zur Höhe des Flüssigkeitspegels 9 mit der Reaktionsflüssigkeit befüllt worden ist, wird erfindungsgemäß im Betrieb über die Umlaufleitung 3 ein Teil der Reaktionsflüssigkeit kontinuierlich entnommen und mindestens einer im oberen Teil des Reaktors angebrachten Düse 5 zugeführt, wozu vorteilhaft eine in die Umlaufleitung 3 integrierte Pumpe 4 verwendet werden kann. Gewünschtenfalls können in die Umlaufleitung 3 auch noch ein oder mehrere Wärmetauscher 12 zur Erwärmung und/oder Kühlung der Reaktionsflüssigkeit eingebaut sein. Die Entnahme der Reaktionsflüssigkeit kann im Prinzip in jeder Höhe des Reaktorkörpers 2 erfolgen, vorzugsweise wird die Reaktionsflüssigkeit einer strömungsberuhigten Zone am unteren Ende des Reaktorkörpers 2 entnommen. Je nach dem Gehalt der Reaktionsflüssigkeit an suspendierten Gasbläschen, kann es zweckmäßig sein, eine Pumpe 4 zu wählen, die in der Lage ist, Gas/Flüssigkeitsmischungen zu pumpen, beispielsweise eine Seitenkanalpumpe.

Der Gehalt der entnommenen Reaktionsflüssigkeit an dispergierten Gasbläschen hängt im allgemeinen vom Ort der Entnahme ab, er ist im allgemeine in den stark durchströmten Bereichen des Reaktors wesentlich höher als in der strömungsberuhigten Zone am unteren Ende des Reaktors, d.h. unterhalb der Prallplatte, wo er praktisch vernachlässigt werden kann.

Die Düse 5 ist im oberen Teil des Reaktorkörpers, vorzugsweise in dem mit Reaktionsgasen ausgefüllten Kopfraum 10, angebracht, so daß sich ihre Düsenspitze im Betriebszustand im Bereich oder unterhalb des Flüssigkeitspegels jedoch oberhalb eines der Düse 5 zugeordneten Leitorgans 6 befindet. Als Düse 5 kann im Prinzip jede beliebige zur Erzeugung eines Flüssigkeitsstrahls geeignete Düsenkonstruktion verwendet werden, in einer besonders bevorzugten Ausführungsform des erfindungsgemäßen Verfahrens ist die Düse 5 als Zweistoffdüse ausgestaltet, mit der das Reaktionsgas durch den in der Düse erzeugten Flüssigkeitsstrahl angesaugt, in diesen eingemischt und mit diesem in der Reaktionsflüssigkeit in Form feiner Gasbläschen dispergiert wird.

Der Düse 5 ist ein oben und unten offenes Leitorgan 6 zugeordnet, das die gleiche Querschnittsform des Reaktorkörpers hat aber auch von dieser verschieden sein kann. Vorzugsweise hat das Leitorgan 6 die gleiche Querschnittsform wie der Reaktorkörper 2, ist vorteilhaft kreisrund Und ist im Falle der einfachsten Ausgestaltung des erfindungsgemäßen Verfahrens bevorzugt im mittiger Position im Innenraum des Reaktorkörpers über an sich herkömmliche Befestigungsvorrichtungen angebracht. Die Form des Leitorgans 6 entspricht der eines offenen Rohres, weshalb es im Folgenden auch als Einsteckrohr bezeichnet wird.

Vorteilhaft ist das Leitorgan 6 bezüglich der Düse 5 im Reaktorkörper so angeordnet, daß sich die Düsenspitze 11 in mittiger, also in ungefähr konzentrischer Position, vorzugsweise in exakt konzentrischer Position, bezüglich der Innenquerschnittsfläche des Leitorgans 6 befindet. Das obere Ende des Einsteckrohres 6 befindet sich vorteilhaft unterhalb des Flüssigkeitspegels 9 der Reaktionsflüssigkeit im Abstand a zur Düsenspitze 11. Der Abstand a kann in Abhängigkeit vom Energieeintrag des von der Düse 5 erzeugten Flüssigkeitsstrahl variiert werden, im allgemeinen beträgt er das 0,1- bis 0,8-fache, vorzugsweise das 0,2- bis 0,4-fache des Reaktorinnendurchmessers. Das untere Ende des Einsteckrohres 6 befindet sich im allgemeinen im Abstand b oberhalb des Reaktorbodens 13 oder im Falle der bevorzugten Verwendung einer Umlenkvorrichtung 7, die beispielsweise als Prallplatte ausgestaltet sein kann, im Abstand b von dieser Umlenkvorrichtung 7. Die Größe des Abstands b kann in weiten Bereichen variiert werden, und sollte lediglich hinreichend groß sein, damit die Flüssigkeitsströmung das Einsteckrohr 7 ungehindert verlassen kann. Im allgemeinen beträgt der Abstand b das 0,15- bis 1,2-fache, vorzugsweise das 0,3- bis 0,6-fache des Reaktorinnendurchmessers.

Die Umlenkvorrichtung 7 wird diese zweckmäßigerweise in einem gewissen Abstand zum Reaktorboden 13 installiert, wobei dieser Abstand im Prinzip beliebig gewählt werden kann. Der Raum zwischen der Umlenkvorrichtung 7 und dem Reaktorboden 13 ist ebenfalls mit Reaktionsflüssigkeit befüllt und steht mit der Reaktionsflüssigkeit oberhalb der Umlenkvorrichtung 7, z.B. über einem Durchlaß zwischen der Umlenkvorrichtung 7 und der Wandung des Reaktorkörpers 2 oder über Durchlässe in der Umlenkvorrichtung 7 in innigen Kontakt. Wird gemäß einer bevorzugten Ausführungsform des erfindungsgemäßen Verfahrens ein Teil der Reaktionsflüssigkeit durch die Umlaufleitung 3 aus einer strömungsberuhigten Zone im unteren Teil des Reaktorkörpers 2 entnommen, so geschieht dies vorteilhaft über eine Entnahmestelle, die sich im Raum zwischen der Umlenkvorrichtung 7 und dem Reaktorboden 13 befindet. Im Falle der Beschickung des Reaktorkörpers 2 mit flüssigem und/oder gasförmigen Reaktanten über Leitung 14 in dem Raum zwischen der Umlenkvorrichtung 7 und dem Reaktorboden 13 kann es vorteilhaft sein, diese Ströme mittels strömungslenkender Einbauten so zu lenken, daß keine unmittelbare Vermischung zwischen dem über Umlaufleitung 3 entnommenen Flüssigkeitsstroms und den über Leitung 14 frisch zugeführten Reaktanten erfolgt.

Das im Einsteckrohr 6 wird so dimensioniert, daß das Verhältnis aus seiner Innenquerschnittsfläche zur gesamten Innenquerschnittsfläche des Reaktorkörpers 2, die sich aus dessen Innendurchmesser D errechnet, im allgemeinen 0,03 bis 0,6, vorzugsweise 0,06 bis 0,4 und besonders bevorzugt 0,1 bis 0,36 beträgt. Im Falle der Verwendung

mehrerer Einsteckrohre 6, wird deren Innenquerschnittsfläche so ausgelegt, daß das Verhältnis aus der Summe der Innenquerschnittsflächen dieser Einsteckrohre 6 zur gesamten Innenquerschnittsfläche des Reaktorkörpers 2 in den vorgenannten Bereichen liegt. Das Verhältnis aus der Länge L des Einsteckrohres 6 zu dessen Innendurchmesser d beträgt im allgemeinen 3 bis 40, vorzugsweise 4 bis 20 und besonders bevorzugt 6 bis 12.

Die gasförmigen Reaktanten, also das Synthesegas und gegebenenfalls gasförmige Olefine, sowie gewünschtenfalls diesen gasförmigen Reaktanten zugemischte aber darin enthaltende Inertgase, als auch die flüssigen Reaktanten, also das Olefin, gegebenenfalls in verflüssigter Form sowie Katalysatorlösung, und gewünschtenfalls Lösungsmittel für die-Hydroformylierungsreaktion können dem Reaktor 1 prinzipiell an einer beliebigen Stelle des Reaktors 1 zugeführt werden. Vorzugsweise werden die gasförmigen Reaktanten unterhalb der Prallplatte 7 (Strom 14) eingeleitet.

Gasförmige Zuströme können z.B. aber auch in den Kopfraum 10, sowie an verschiedenen Stellen entlang des Reaktors eingeleitet werden wie dies durch die Ströme 15 und 16 angedeutet ist. Dies gewinnt insbesondere dann an Bedeutung wenn es sich bei den gasförmigen Strömen um Strippgas handelt.

Denkbar ist auch die Einleitung eines Gasstroms, insbesondere wenn es um Gase handelt die an der Reaktion beteiligt sind, über eine Zweistoffdüse. Das gleiche gilt für flüssige Zuströme. Bevorzugte Stellen sind hier Strom 20 bzw. die Zugabe über eine Zweistoffdüse (Strom 18).

Die flüssige Phase wird dem Reaktor bevorzugt als Teilstrom (Strom 21) aus dem Pumpenkreislauf (3) entnommen. Andere Stellen sind aber auch denkbar. Die gasförmige Phase wird dem Reaktor bevorzugt aus dem Kopfraum (10) über Strom (11) entnommen. Andere Möglichkeiten, z.B. unterhalb der Prallplatte (17), sind ebenfalls denkbar.

Im einzelnen geht man z.B. bei Betrieb des erfindungsgemäß zu verwendenden Reaktors so vor, daß es für die Inbetriebnahme soweit mit Flüssigkeit gefüllt wird, bis der Flüssigkeitsspiegel die Düse (5) berührt. Durch den über die Düse (5) mit hoher Geschwindigkeit eingebrachten externen Kreislaufstrom wird in dem Reaktor eine Umlaufströmung erzeugt. Die Strömung ist im Einsteckrohr (6) nach unten gerichtet und im Spalt zwischen dem Einsteckrohr und der Reaktoraußenwand (8) nach oben. Die Umlenkung findet an de Prallplatte (17) statt. Die Strömungsgeschwindigkeit im Ringspalt wird über die Druckmeßstellen P/2 und P/3, P/4 erfaßt. Solange noch kein Gas im Reaktor dispergiert ist, stellt sich an den Druckmeßstellen P/1 und P/2 der maximale Differenzdruck ein.

Wenn über das Ventil (22) Flüssigkeit aus dem Reaktor abgelassen wird, bleibt der Füllstand H konstant. Das Volumen der abgelassenen Flüssigkeit wird lediglich durch Gas ersetzt. Das Gas wird über die Düse aus dem Kopfraum angesaugt und durch die abwärtsgerichtete Strömung mitgerissen. Das auf diese Weise im Einsteckrohr nach unten geförderte Gas steigt im Spaltraum wieder nach oben. Die Düse (5) saugt solange Gas aus dem Kopfraum an, bis der Flüssigkeitsspiegel den Düsenmund berührt. Der Reaktor ist damit in der Lage intern Gas im Kreislauf zu fördern. Die Prallplatte (17) unterdrückt dabei das Mitreißen von Gasblasen in den externen Pumpenkreislauf.

Umgekehrt kann durch die erneute Zufuhr von Flüssigkeit in den Reaktor das Gas wieder vollständig aus der Umlaufströmung verdrängt werden. Auf diese Weise ist eine Regelung des Gasgehaltes möglich. Für die Regelung kann z.B. der Gasgehalt im Außenraum (8), der über die Druckmeßstellen P/1 und P/2 zugänglich ist, herangezogen werden.

Voraussetzung für das Zustandekommen der zweiphasigen Umlaufströmung ist, daß die Strömungsgeschwindigkeit im Einsteckrohr größer ist als die Aufstiegsgeschwindigkeit der dispergierten Gasphase.

Der intern umgewälzte Flüssigkeitsstrom ist um ein Vielfaches größer als der Volumenstrom des Treibstrahls. Ebenso kann der intern geförderte Kreisgasstrom ein Vielfaches der von außen zugeführten Gasströme betragen. Konzentrations- und Temperaturgradienten in der flüssigen Phase werden dadurch sehr stark reduziert. Der Reaktor kann somit im gesamten Volumen unter den zur Erzielung einer maximalen Selektivität günstigsten Bedingungen betrieben werden.

Obgleich das Prinzip des erfindungsgemäß zu verwendenden Reaktors (Zusammenfassend in Ullmann's Encyclopedia of Industrial Chemistry Vol. B4 S. 298 (1992) bekannt war, gingen alle technischen Lösungen bei der Hydroformylierung bisher in eine andere Richtung, nämlich zur Verwendung von Rührkesseln und Blasensäulenreaktoren. Offenbar bestand ein Vorurteil gegen die Verwendung von "Jet-Loop-Reaktoren" der hier beschriebenen Art. Es war in der Tat bei größeren Reaktoren zu befürchten, daß über den Strahl nicht genügend Gas eingetragen werden kann. Aber auch der umgekehrte Fall, daß sich zu große, nicht regelbare Gasgehalte einstellen, war zu befürchten. Die Vorbehalte gegen diese Art von Reaktoren waren vor allem auch deshalb groß, weil die genannten unerwünschten Effekte sehr stark von den Stoffeigenschaften (dem Koaleszenzverhalten) der Gas-/Flüssigkeitsmischung abhängig sind.

Entgegen diesem Vorurteil bietet der erfindungsgemäß zu verwendende Reaktor eine Reihe von besonders für die Hydroformylierung wichtigen Vorteilen wie:

- Einstellbarer und regelbarer Gasgehalt
- hohe Raumzeitausbeute

- große interne Umwälzströme und damit flache Gradienten
- keine exteren Gasumwälzung (Kreisgaskompressor)
- Entkopplung von Gasgehalt und Feedgasströmen
- große Stoffübertragungsgeschwindigkeit zwischen Flüssigkeit und Gas
- hohe Leistungseinträge möglich
- einfache Kühlung über externen Kreislauf
- hoher CO-Umsatz

Die Reaktionsbedingungen der Hydroformylierung bzw. der aminierenden Hydroformylierung sind aus der Fachliteratur wohlbekannt. Im einzelnen wird auf B. Cornils in J. Falbe, New Syntheses with Carbon Monoxide, Springer Verlag, Berlin [1980] S. 1-181, verwiesen. In der Regel wird die erfindungsgemäß auszuführende Hydroformylierung bzw. aminierende Hydroformylierung wie bei M. Beller, B. Cornils, C.D. Frohning, C.W. Kohlpainter, J. Mol. Catal. A, 104 (1995) 17-85, beschrieben vorgenommen.

Als Edukte kommen beliebige, hydroformylierbare Olefine in Betracht, insbesondere aliphatische Olefine mit 2 bis 20 C-Atomen, bevorzugt $\alpha$-Olefine oder interne lineare Olefine mit 2 bzw. 4 bis 20 C-Atomen.

Beispiele

A) Versuchsanordnung

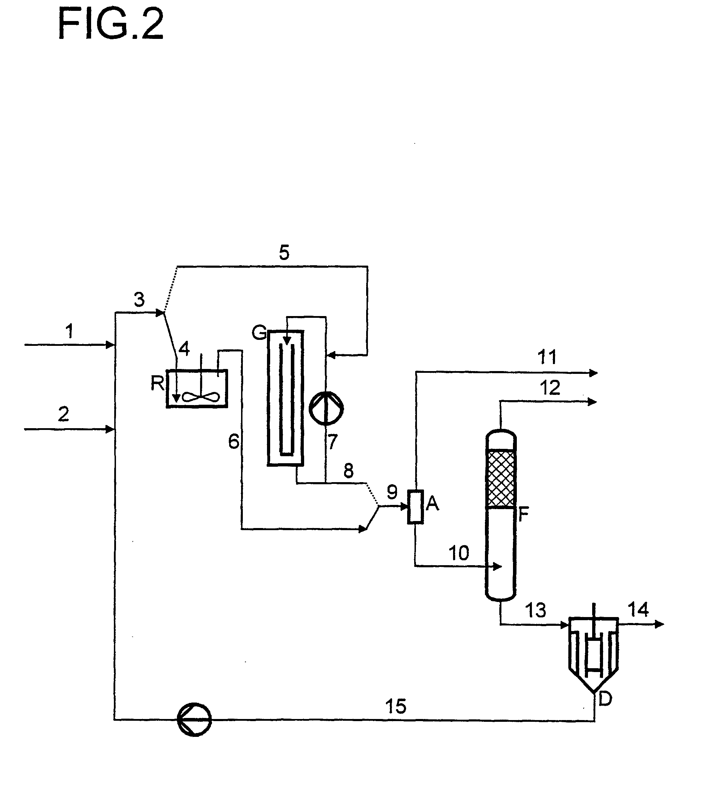

Für die Versuche der folgenden Beispiel wurde eine kontinuierliche Miniplantanlage benutzt, die in Fig. 2 schematisch dargestellt ist und im folgenden erläutert wird. Selbstverständliche Anlagendetails, die zur Veranschaulichung des erfindungsgemäßen Verfahrens nicht erforderlich sind, wurden aus Gründen der Übersichtlichkeit nicht in Fig. 2 aufgenommen.

Die Anlage konnte wahlweise mit einem mechanisch gerührten Autoklaven (R) als Reaktor oder mit einem erfindungsgemäß zu verwendenden Reaktor (G) betrieben werden. Der Autoklav verhält sich wie in ideal durchmischter Rührkesselreaktor.

Über die Leitung 1 und 2 wird Olefin bzw. $CO/H_2$ Gemisch in den Kalysator + Hochsiederrückführungsstrom eingespeist und in Leitung (3) alternativ entweder über Leitung (5) in den erfindungsgemäß zu verwendenden Gasumlaufreaktor (G) oder in den Rührkesselreaktor (R) eingeleitet.

Der Hydroformylierungsaustrag aus dem Reaktor wurde über Leitung (6/9 bzw. (8/9) nach Entspannung und Abtrennung der flüssigen Phase von überschüssigem Synthesegas im Druckabschneider (A) über Leitung 10 einer destillativen Aufarbeitung (bestehend aus Flash (F) und Sambayverdampfer (D)) zugeführt. Der Sumpf aus der destillativen Abtrennung wurde über Leitung 15 in die Hydroformylierungsstufe zurückgeführt. Über die Leitungen 11 wird das Abgas über die Leitung 12 die nicht reagierten Olefine und über die Leitung 14 die entstandenen Aldehyde abgezogen. Diese Verschaltung hat sich im experimenteller Hinsicht als günstig erwiesen; andere Durchführungen einer Hydroformylierung mit Hilfe des erfindungsgemäß zu verwendenden Reaktors sind selbstverständlich nicht ausgeschlossen.

Beispiel 1

Ein kontinuierlicher Versuch wurde in einem erfindungsgemäß zu verwendenden Gasumlaufreaktor (G) mit einem Gesamtvolumen von 4,35 l durchgeführt. Auf den Gasumlaufreaktor entfallen dabei 3,6 l, auf den Umpumpkreislauf 0,75 l. Aufgrund der großen Kreislaufmenge muß der Umpumpkreislauf mit zum aktiven Reaktorvolumen gezählt werden. Nach Abtrennung von überschüssigem Olefin in dem Flashverdampfer (F) und Abtrennung des Reaktionsproduktes über einen Wischblattverdamper (D) wurde der katalysatorhalte Sumpf über Leitung 15 in den Reaktor zurückgeführt. Die Rhodiumkonzentration im Reaktor betrug ca. 120 ppm. Das Ligand/Rhodium-Verhältnis betrug 120:1 (mol/mol). Die unter den Reaktionsbedingungen entstandenen Hochsieder wurden als Lösungsmittel verwendet. $CO/H_2$ wurde im molaren Verhältnis 1:1 eingesetzt. Der Druck (20bar) und die Temperatur (105°C) wurden konstant gehalten.

Eine Kreislaufmenge von 145 kg/h wurde mit Hilfe einer Pumpe durch Leitung (7) über eine Düse mit 1,8 mm Durchmesser in den Reaktor (G) gefördert. Der Gasanteil im Reaktor wurde auf 10 % eingestellt, das entspricht einem Gasvolumen von 0,345 l, und konstant gehalten. Durch Variation des $CO/H_2$-Verhältnisses im Frischgas wurde die erwünschte Selektivität zum linearen Isomer erreicht.

Die Belastung betrug 450 g/(h Propylen (99,3 %ig, Rest Propan). Nach Anfahren der Anlage wurde eine Propylenumsatz von 84 % mit einer Selektivität zu Aldehyden von 95 % bei einem n-Anteil von 87 % erreicht. Die RZA betrug bezogen auf das Gesamtvolumen 140,5 g/(h).

Beispiel 2

Bei einem weiteren kontinuierlichen Versuch im Gasumlaufreaktor (G) und Abtrennung von überschüssigem Olefin in dem Flashverdampfer (F) und Abtrennung des Reaktionsprodukts über einem Sambay-Verdampfer (D) wurde der katalysatorhaltige Sumpf in den Reaktor zurückgefahren. Die Rhodiumkonzentration im Reaktor betrug ca. 120 ppm. Das Ligand/Rhodium-Verhältnis betrug 120:1 (mol/mol). Die unter den Reaktionsbedingungen entstandenen Hochsieder wurden als Lösungsmittel verwendet. $CO/H_2$ wurde im Verhältnis 1:1 eingesetzt. Der Druck (20 bar) und die Temperatur (105°C) wurden konstant gehalten.

Eine Kreislaufmenge von 145 kg/h wurde mit Hilfe einer Pumpe durch Leitung (7) in den Reaktor gefördert. Der Gasanteil im Reaktor wurde auf 2 % eingestellt und konstant gehalten. Das Gasvolumen betrug damit nur etwa 0,07 l. Der auf das Gesamtvolumen bezogene Gasgehalt war damit um ca. 6 % kleiner als im Beispiel 1. Durch Variation des $CO/H_2$-Verhältnisses im Frischgas wurde die erwünschte Selektivität zum linearen Isomer erreicht.

Die Belastung betrug 450g/h Propylen (99,3 %ig, Rest Propan). Nach Anfahren der Anlage wurde im Rahmen der Meßgenauigkeit der gleiche Propylenumsatz wie in Beispiel 1 gemessen und mit 95 % auch die gleiche Selektivität zu Aldehyden. Trotz des geringeren Gasgehalts, änderte sich der CO-Partialdruck nicht und der n-Anteil von 87 % wurde wieder erreicht.

Beispiel 3 (Vergleich)

Ein kontinuierlicher Versuch wurde in einem 2,5-1-Hubrührautoklaven mit Innenkühlung (verfügbares Flüssigkeitsvolumen 1,7 l) durchgeführt. Nach Abtrennung von überschüssigem Olefin in dem Flash und Abtrennung des Reaktionsprodukts über einem Sambay-Verdampfer wurde der katalysatorhaltige Sumpf in den Reaktor zurückgefahren. Die Rhodiumkonzentration im Reaktor betrug ca. 100 ppm. Das Ligand/Rhodium-Verhältnis betrug 120:1 (mol/mol). Die unter den Reaktionsbedingungen entstandenen Hochsieder wurden als Lösungsmittel verwendet. $CO/H_2$ im Verhältnis 1:1 wurde eingesetzt, der Druck (20 bar) und die Temperatur (105°C) wurden konstant gehalten.

Die Belastung betrug 250 g/h Propen (99,3 %ig, Rest Propan). Nach Anfahren der Anlage wurde ein Propylenumsatz von 80 % mit einer Selektivität zu Aldehyden von 92 % bei einem n-Anteil von 88 % erreicht. Der RZA betrug wegen des großen, nicht regelbaren Gasgehaltes nur 125 g(1h).

## Patentansprüche

1. Verfahren zur Herstellung von Aldehyden und/oder Alkoholen oder Aminen durch die Umsetzung von Olefinen in flüssiger Phase mit Kohlenmonoxid und Wasserstoff, wobei ein Teil dieser Gase in Form von Gasblasen in der Reaktionsflüssigkeit dispergiert und ein anderer Teil in der Reaktionsflüssigkeit gelöst ist, in An- oder Abwesenheit eines primären oder sekundären Amins und in Gegenwart von homogen in der Reaktionsflüssigkeit gelösten Kobalt-, Rhodium-, Palladium- oder Ruthenium-Carbonylkomplexen mit einem Phosphor-, Arsen-, Antimon- oder Stickstoff enthaltenden Liganden bei erhöhter Temperatur und bei einem Druck von 1 bis 100 bar, **dadurch gekennzeichnet, daß** man die Umsetzung in einem senkrecht angeordneten Rohrreaktor, umfassend einen Reaktorkörper und mindestens eine Umlaufleitung, vornimmt, einen Teil der Reaktionsflüssigkeit kontinuierlich über die Umlaufleitung mindestens einer im oberen Teil des Reaktorkörpers angebrachten Düse zuführt, der ein oben und unten offenes, durch parallele Wände begrenztes Leitorgan im Inneren des Reaktors sowie eine unterhalb der unteren Öffnung des Leitorgans befindlichen Prallplatte zugeordnet ist, und mit dieser Düse in diesem Leitorgan einen abwärts gerichteten dispergierte Gasblasen enthaltenden Flüssigkeitsstrom erzeugt, der nach Verlassen des Leitorgans in eine im Zwischenraum zwischen der Wandung des Leitorgans und der Wandung des Reaktorkörpers aufwärts fließenden Strom umgelenkt wird, und am oberen Ende des Leitorgans vom Strahl der dem Leitorgan zugeordneten Düse in das Leitorgan gesaugt wird.

2. Verfahren nach Anspruch 1, **dadurch gekennzeichnet, daß** man die Hydroformylierung in einem Reaktor 1, umfassend den Reaktorkörper 2 und mindestens eine Umlaufleitung 3 sowie Zuleitungs- und Ableitungsvorrichtungen für die Reaktanten und den Reaktionsaustrag durchführt und aus dem Reaktorkörper 2 über mindestens eine Umlaufleitung 3 kontinuierlich einen Teil der Reaktionsflüssigkeit entnimmt und mindestens einer im oberen Teil des Reaktorkörpers 2 befindlichen Düse 5 zuführt, und dadurch mindestens einen Strahl erzeugt, der in mindesten einen im Reaktor angebrachten Leitorgan 6 eine abwärts gerichtete Strömung erzeugt, die nach Auftreffen auf mindestens eine dem betreffenden Leitorgan oder den Leitorganen 6 zugeordnete Umlenkvorrichtung 7 in Form einer Prallplatte in eine im Zwischenraum 8 zwischen Reaktorkörper 2 und dem betreffenden Leitorgan 6 aufwärts fließende Strömung umgelenkt wird, wobei in dem Reaktorkörper 2 mindestens ein Leitorgan 6 so angebracht ist, daß

a) dessen oberes Ende im Betriebszustand unterhalb des Flüssigkeitspegels 9 der im Reaktorkörper 2 befindlichen Reaktionsflüssigkeit liegt und mindestens eine diesem Leitorgan 6 zugeordnete Düse 5 sich in etwa konzentrischer Position bezüglich der Querschnittsfläche dieses Leitorgans 6 befindet,

b) dessen unteres Ende im Abstand über dem Reaktorboden 13 oder über mindestens einer dem Leitorgan 6 zugeordneten Umlenkvorrichtung 7 gelegen ist, und das oder die Leitorgane 6 so dimensioniert sind, daß das Verhältnis aus der Innenquerschnittsfläche des oder der Summe der Innenquerschnittsflächen der Leitorgane 6 und der gesamten Innenquerschnittsfläche des Reaktorkörpers 2 0,03 bis 0,6 und das Verhältnis aus der Länge L mindestens eines einer Düse 5 zugeordneten Leitorgans 6 und dessen Innendurchmesser d 3 bis 40 beträgt und die mindestens eine Düse 5 im Kopfraum 10 des Reaktorkörpers 2 so angeordnet ist, daß deren Spitze 11 sich im Betriebszustand des Reaktors im Bereich oder unterhalb des Flüssigkeitspegels 8, jedoch im Abstand a, der das 0,1- bis 0,8-fache des Reaktorinnendurchmessers beträgt, vom oberen Ende des dieser Düse 5 zugeordneten Leitorgans 6 befindet.

**3.** Verfahren nach den Ansprüchen 1 und 2, dadurch gekennzeichnet, daß ein Reaktor 1 verwendet wird, in dessen Reaktorkörper 2 ein einziges Leitorgan 6 angebracht ist.

**4.** Verfahren nach den Ansprüchen 1 bis 3, **dadurch gekennzeichnet, daß** man einen Reaktor 1 verwendet, in dem mindestens eine Umlaufleitung 3 und eine Vorrichtung zur Wärmeübertragung 12 enthalten ist.

**5.** Verfahren nach den Ansprüchen 1 bis 4, **dadurch gekennzeichnet, daß** man die Reaktionsflüssigkeit aus dem unteren Teil der Reaktorkörper 2 entnimmt und über mindestens eine Umlaufleitung 3 der Düse 5 zuführt.

**6.** Verfahren nach den Ansprüchen 1 bis 5, **dadurch gekennzeichnet, daß** man einen Reaktor 1 verwendet, in dem zur Umlenkung des aus mindestens einem Leitorgan 6 kommenden Flüssigkeitsstrahls im Abstand b, der das 0,15- bis 1,2-fache des Reaktorinnendurchmessers beträgt, vom unteren Ende eines Leitorgans 6 mindestens eine Umlenkvorrichtung 7 in Form einer Prallplatte eingebaut ist und sich unterhalb der Prallplatte ein Beruhigungsraum zur Entnahme des Flüssigkeitsstroms für die Umlaufleitung befindet.

**7.** Verfahren nach den Ansprüchen 1 bis 6, **dadurch gekennzeichnet. daß** man einen Reaktor 1 verwendet, dessen Düse oder Düsen 5 als Zweistoffdüsen ausgestaltet sind.

**8.** Verfahren nach den Ansprüchen 1 bis 7, **dadurch gekennzeichnet, daß** man ein Olefin in Gegenwart eines homogen in der Reaktionsflüssigkeit gelösten Komplexes aus einer Kobaltcarbonylverbindung und einem phosphorhaltigen Liganden als Katalysator bei einer Temperatur von 160 bis 200°C und einem Druck von 50 bis 100 bar zu dem entsprechenden Aldehyd oder Alkohol oder Alkohol-/Aldehyd-Gemisch hydrierend hydroformyliert.

**9.** Verfahren nach den Ansprüchen 1 bis 7, **dadurch gekennzeichnet, daß** man ein Olefin in Gegenwart eines Rhodiumoder Ruthenium-Carbonylkomplexes mit einem phosphor-, arsenoder antimon enthaltenden Liganden als Katalysator bei einer Temperatur von 60 bis 130°C und bei einem Druck von 1 bis 50 bar zu dem entsprechenden Aldehyd hydroformyliert.

**10.** Verfahren nach den Ansprüchen 1 bis 7, **dadurch gekennzeichnet, daß** man ein Olefin in Gegenwart eines homogen im Reaktionsmedium gelösten Rhodium-, Palladium- oder Rutheniumcarbonylkomplexes mit einem phosphor-, arsen- oder antimonenthaltenden Liganden als Katalysator und in Gegenwart von einem primären $C_1$- bis $C_{12}$-Amin oder einem sekundären $C_2$- bis $C_{24}$-Amin zu den entsprechenden primären, sekundären oder tertiären Amin bei einer Temperatur von 60 bis 150°C und bei einem Druck von 1 bis 50 bar aminierend hydroformyliert.

## Claims

**1.** A process for the preparation of aldehydes and/or alcohols or amines by reacting olefins in the liquid phase with carbon monoxide and hydrogen, a part of these gases being dispersed in the form of gas bubbles in the reaction liquid and another part being dissolved in the reaction liquid, in the presence or absence of a primary or secondary amine and in the presence of a cobalt carbonyl, rhodium carbonyl, palladium carbonyl or ruthenium carbonyl complex dissolved homogeneously in the reaction liquid and having a phosphorus-, arsenic-, antimony- or nitrogen-containing ligand, at elevated temperatures and at 1 to 100 bar, wherein the reaction is carried out in a vertically arranged, tubular reactor comprising a reactor body and at least one circulation line, a part of the reaction liquid

is fed continuously via the circulation line to at least one nozzle which is mounted in the upper part of the reactor body and is coordinated with a guide member, open at the top and bottom and bounded by parallel walls, in the interior of the reactor and with a baffle present below the lower opening of the guide member, and a downward-directed liquid stream containing dispersed gas bubbles is produced by means of this nozzle in this guide member and, after leaving the guide member, is deflected into a stream flowing upward in the space between the wall of the guide member and the wall of the reactor body and is sucked into the guide member at the upper end of the guide member by the jet of the nozzle coordinated with the guide member.

**2.** A process as claimed in claim 1, wherein the hydroformylation is carried out in a reactor 1, comprising the reactor body 2 and at least one circulation line 3 as well as feed and discharge apparatuses for the reactants and the reaction discharge, and a part of the reaction liquid is removed continuously from the reactor body 2 via at least one circulation line 3 and fed to at least one nozzle 5 present in the upper part of the reactor body 2, thus producing at least one jet which generates, in at least one guide member 6 mounted in the reactor, a downward-directed stream which, after meeting at least one deflection apparatus 7 coordinated with the relevant guide member or with the guide members 6 and in the form of a baffle, is deflected into a stream flowing upward in the space 8 between reactor body 2 and the relevant guide member 6, at least one guide member 6 being mounted in the reactor body 2 so that

a) in the operating state, the upper end of said guide member is below the liquid level 9 of the reaction liquid present in the reactor body 2 and at least one nozzle 5 coordinated with this guide member 6 is present in the roughly concentric position relative to the cross-sectional area of this guide member 6,

b) the lower end of said guide member is located a distance above the reactor base 13 or above at least one deflection apparatus 7 coordinated with the guide member 6, and the guide member or members 6 is or are dimensioned such that the ratio of the internal cross-sectional area of the guide member 6 or of the sum of the internal cross-sectional areas of the guide members 6 to the total internal cross-sectional area of the reactor body 2 is from 0.03 to 0.6 and the ratio of the length L of at least one guide member 6 coordinated with a nozzle 5 to the internal diameter d of said guide member is from 3 to 40 and the at least one nozzle 5 is arranged in the top space 10 of the reactor body 2 in such a way that, in the operating state of the reactor, the tip 11 of said nozzle is in the region of or below the liquid level 9 but a distance a, which is from 0.1 to 0.8 times the internal diameter of the reactor, from the upper end of the guide member 6 coordinated with this nozzle 5.

**3.** A process as claimed in claims 1 and 2, wherein the reactor 1 used is one in whose reactor body 2 a single guide member 6 is mounted.

**4.** A process as claimed in any of claims 1 to 3, wherein the reactor 1 used is one which contains at least one circulation line 3 and an apparatus for heat transfer 12.

**5.** A process as claimed in any of claims 1 to 4, wherein the reaction liquid is removed from the lower part of the reactor body 2 and fed via at least one circulation line 3 to the nozzle 5.

**6.** A process as claimed in any of claims 1 to 5, wherein the reactor 1 used is one in which, for deflecting the liquid jet from at least one guide member 6, at least one deflection apparatus 7 in the form of a baffle is installed a distance b, which is from 0.15 to 1.2 times the internal diameter of the reactor, from the lower end of a guide member 6 and a calming space for removing the liquid stream for the circulation line is present below the baffle.

**7.** A process as claimed in any of claims 1 to 6, wherein the reactor 1 used is one whose nozzle or nozzles 5 is or are designed as a binary nozzle or nozzles.

**8.** A process as claimed in any of claims 1 to 7, wherein an olefin is subjected to hydroformylation under hydrogenating conditions in the presence of a complex dissolved homogeneously in the reaction liquid and comprising a cobalt carbonyl compound and a phosphorus-containing ligand as catalyst at from 160 to 200°C and from 50 to 100 bar to give the corresponding aldehyde or alcohol or alcohol/aldehyde mixture.

**9.** A process as claimed in any of claims 1 to 7, wherein an olefin is hydroformylated in the presence of a rhodium carbonyl or ruthenium carbonyl complex having a phosphorus-, arsenic- or antimony-containing ligand as catalyst at from 60 to 130°C and from 1 to 50 bar to give the corresponding aldehyde.

**10.** A process as claimed in any of claims 1 to 7, wherein an olefin is hydroformylated under aminating conditions in the presence of a rhodium carbonyl, palladium carbonyl or ruthenium carbonyl complex dissolved homogeneously in the reaction medium and having a phosphorus-, arsenic- or antimony-containing ligand as catalyst and in the presence of a primary $C_1$- to $C_{12}$-amine or of a secondary $C_2$- to $C_{24}$-amine to give the corresponding primary, secondary or tertiary amine at from 60 to 150°C and from 1 to 50 bar.

## Revendications

**1.** Procédé pour la fabrication d'aldéhydes et/ou d'alcools ou d'amines par la transformation d'oléfines en phase liquide avec du monoxyde de carbone et de l'hydrogène, dans lequel une partie de ces gaz est dispersée sous forme de bulles de gaz dans le liquide réactionnel et une autre partie est dissoute dans le liquide réactionnel, en présence ou en l'absence d'une amine primaire ou secondaire et en présence de complexes carbonyliques de cobalt, rhodium, palladium ou ruthénium dissous de façon homogène dans le liquide réactionnel, avec un ligand contenant du phosphore, de l'arsenic, de l'antimoine ou de l'azote, à température relevée et à une pression de 1 à 100 bar, **caractérisé en ce que** l'on effectue la transformation dans un réacteur tubulaire disposé verticalement, comprenant un corps de réacteur et au moins une conduite de circulation, on amène une partie du liquide réactionnel en continu, par la conduite de circulation au moins à une buse disposée dans la partie supérieure du corps de réacteur à laquelle sont affectés un organe de guidage ouvert en haut et en bas, limité par des parois parallèles à l'intérieur du réacteur, ainsi qu'une plaque déflectrice située en dessous de l'ouverture inférieure de l'organe de guidage, et **en ce qu'**avec cette bues on produit dans cet organe de guidage un écoulement de liquide dirigé vers le bas contenant des bulles de gaz dispersées qui, après avoir quitté l'organe de guidage, est dévié en un courant s'écoulant vers le haut dans l'espace intermédiaire entre la paroi de l'organe de guidage et la paroi du corps de réacteur, et à l'extrémité supérieure de l'organe de guidage, une aspiration du jet de la buse affectée à l'organe de guidage est effectuée dans l'organe de guidage.

**2.** Procédé selon la revendication 1, **caractérisé en ce que** l'on effectue l'hydroformylation dans un réacteur 1, comprenant le corps de réacteur 2 et au moins une conduite de circulation 3 ainsi que des dispositifs d'amenée et de dérivation pour les réactifs et le produit de la réaction, et l'on prélève du corps de réacteur 2, par au moins une conduite de circulation 3, en continu une partie du liquide réactionnel et on l'amène au moins à une buse 5 située dans la partie supérieure du corps de réacteur 2, et **en ce que** l'on produit au moins un jet qui produit un écoulement dirigé vers le bas dans au moins un organe de guidage 6 monté à l'intérieur du réacteur, lequel, après impact sur au moins un dispositif de déviation 7 en forme de plaque déflectrice, affecté à l'organe de guidage correspondant ou aux organes de guidage 6, est dévié en un courant s'écoulant vers le haut dans l'espace intermédiaire 8 entre corps de réacteur 2 et organe de guidage 6 correspondant, au moins un organe de guidage 6 étant disposé dans le corps de réacteur 2 de façon que

a) son extrémité supérieure, à l'état de fonctionnement, se situe en dessous du niveau 9 du liquide réactionnel situé dans le corps de réacteur 2 et au moins une buse 5 affectée à cet organe de guidage 6 se trouve en position approximativement concentrique par rapport à la surface de la section de cet organe de guidage 6,

b) son extrémité inférieure est située à distance, au-dessus du fond du réacteur 13 ou au-dessus d'au moins d'un dispositif de déviation 7 affecté à l'organe de guidage 6, et l'organa ou les organes de guidage 6 sont dimensionnés de façon que le rapport entre la surface interne de la section de l'organe de guidage 6 ou de la somme des surfaces internes des sections des organes de guidage 6 et la surface totale interne de la section du corps de réacteur 2 soit de 0,03 à 0,6 et le rapport entre la longueur L d'au moins un organe de guidage 6 affecté à une buse 5 et son diamètre interne d soit de 3 à 40, et qu'au moins une buse 5 soit disposée dans l'espace de tête 10 du corps de réacteur 2 de façon que son sommet 11 se trouve, à l'état de fonctionnement du réacteur, dans la zone ou en dessous du niveau de liquide 8, mais à la distance a, qui est égale à 0,1 à 0,8 fois le diamètre interne du réacteur, de l'extrémité supérieure de cet organe de guidage 6 affecté à cette buse 5.

**3.** Procédé selon les revendications 1 et 2, **caractérisé en ce qu'**un réacteur 1 est utilisé, dans le corps de réacteur 2 duquel un seul organe de guidage 6 est disposé.

**4.** Procédé selon les revendications 1 à 3, **caractérisé en ce que** l'on utilise un réacteur 1 dans lequel au moins une conduite de circulation 3 et un dispositif pour transfert thermique 12 sont contenus.

5. Procédé selon les revendications 1 à 4, **caractérisé en ce que** l'on prélève le liquide réactionnel de la partie inférieure du corps de réacteur 2 et on l'amène à la buse 5 par au moins une conduite de circulation 3.

6. Procédé selon les revendications 1 à 5, **caractérisé en ce que** l'on utilise un réacteur 1 dans lequel est monté pour la déviation du jet de liquide provenant d'au moins un organe de guidage 6, à la distance b de l'extrémité inférieure d'un organe de guidage 6, qui est égale à 0,15 à 1,2 fois le diamètre interne du réacteur, au moins un dispositif de déviation 7 en forme d'une plaque déflectrice et, en dessous de la plaque déflectrice, se trouve un espace de stabilisation pour le prélèvement de l'écoulement de liquide pour la conduite de circulation.

7. Procédé selon les revendications 1 à 6, **caractérisé en ce que** l'on utilise un réacteur 1 dont la buse ou les buses 5 sont conçues en tant que buses binaires.

8. Procédé selon les revendications 1 à 7, **caractérisé en ce que** l'on hydroformyle, avec hydrogénation, une oléfine en présence d'un complexe constitué d'une combinaison carbonylique de cobalt et d'un ligand contenant du phosphore, dissous de façon homogène dans le liquide réactionnel, en tant que catalyseur à une température de 160 à 200°C et à une pression de 50 à 100 bar, en l'aldéhyde ou l'alcool ou le mélange alcool/aldéhyde correspondant.

9. Procédé selon les revendications 1 à 7, **caractérisé en ce que** l'on hydroformyle une oléfine, en présence d'un complexe carbonylique de rhodium ou ruthénium avec un ligand contenant du phosphore, de l'arsenic ou de l'antimoine en tant que catalyseur à une température de 60 à 130°C et à une pression de 1 à 50 bar, en l'aldéhyde correspondant.

10. Procédé selon les revendications 1 à 7, **caractérisé en ce que** l'on hydroformyle, avec amination, une oléfine en présence d'un complexe carbonylique de rhodium, palladium ou ruthénium dissous de façon homogène dans le milieu de réaction, avec un ligand contenant du phosphore, de l'arsenic ou de l'antimoine, en tant que catalyseur et en présence d'une amine $C_2$ à $C_{12}$ primaire ou d'une amine $C_2$ à $C_{24}$ secondaire, en l'amine primaire, secondaire ou tertiaire correspondante, à une température de 60 à 150°C et à une pression de 1 à 50 bar.

FIG.1

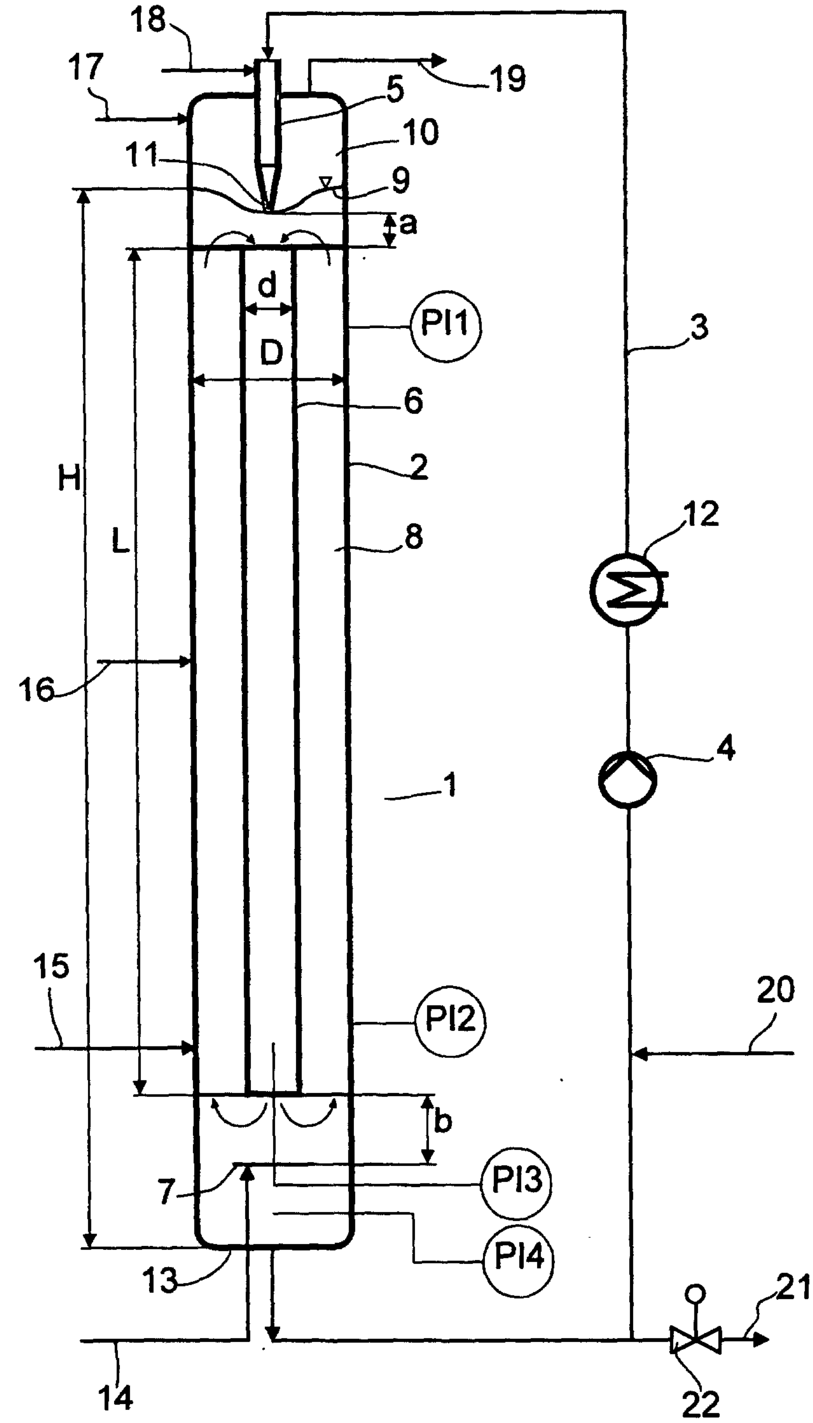
18
17
5
19
10
11
9
a
d
PI1
D
3
6
2
H
8
L
12
16
4
1
15
20
PI2
b
PI3
7
PI4
13
21
14
22

FIG.2